# EUROPÄISCHE PATENTSCHRIFT

(11) **EP 2 171 465 B1**
(45) Veröffentlichungstag und Bekanntmachung des Hinweises auf die Patenterteilung: **21.12.2011**
(21) Anmeldenummer: 08801060.8
(22) Anmeldetag: 28.07.2008
(51) Int. Cl.: G01N 33/574

(54) **LÖSLICHES CADHERIN 17 FÜR DIE DIAGNOSE UND RISIKOSTRATIFIZIERUNG VON KREBS UND TUMOR DES GASTROINTESTINALTRAKTES**
SOLUBLE CADHERIN 17 FOR THE DIAGNOSIS AND RISK STRATIFICATION OF CANCER AND TUMOUR OF THE GASTROINTESTINAL TRACT
CADHÉRINE-17 SOLUBLE POUR LE DIAGNOSTIC ET LA STRATIFICATION DU RISQUE DE CANCER ET DE TUMEUR DU TRACTUS GASTRO-INTESTINAL

(30) Priorität: 26.07.2007 DE 102007034993
(43) Veröffentlichungstag der Anmeldung: 07.04.2010
(73) Patentinhaber: Meyer, Helmut E., 45661 Recklinghausen (DE); Diehl, Hanna, 44789 Bochum (DE); Klein-Scory, Susanne, 58452 Witten (DE); Schmiegel, Wolff, 44797 Bochum (DE); Schwarte-Waldhoff, Irmgard, 40822 Mettmann (DE); Stühler, Kai, 44787 Bochum (DE); Weiss, Jakob, 44866 Bochum (DE)
(72) Erfinder: Meyer, Helmut E., 45661 Recklinghausen (DE); Diehl, Hanna, 44789 Bochum (DE); Klein-Scory, Susanne, 58452 Witten (DE); Schmiegel, Wolff, 44797 Bochum (DE); Schwarte-Waldhoff, Irmgard, 40822 Mettmann (DE); Stühler, Kai, 44787 Bochum (DE); Weiss, Jakob, 44866 Bochum (DE)
(74) Vertreter: Simandi, Claus
(86) Internationale Anmeldenummer: PCT/DE2008/001220
(87) Internationale Veröffentlichungsnummer: WO 2009/012773

(56) Entgegenhaltungen:
- WO-A-2005/110338
- WO-A-2007/141280
- WO-A-2008/026008
- KO S ET AL: "Overexpression of LI-cadherin in gastric cancer is associated with lymph node metastasis" 25. Juni 2004 (2004-06-25), BIOCHEMICAL AND BIOPHYSICAL RESEARCH COMMUNICATIONS, ACADEMIC PRESS INC. ORLANDO, FL, US, PAGE(S) 562 - 568 , XP004512473 ISSN: 0006-291X Seite 567, linke Spalte, Absatz 2
- CHAN ANNIE ON ON ET AL: "Early prediction of tumor recurrence after curative resection of gastric carcinoma by measuring soluble E-cadherin." 15. August 2005 (2005-08-15), CANCER 15 AUG 2005, VOL. 104, NR. 4, PAGE(S) 740 - 746 , XP002505076 ISSN: 0008-543X das ganze Dokument

## Beschreibung

Die Erfindung betrifft ein Verfahren zur *in vitro* Diagnose und Risikostratifizierung von Krebs und Tumoren des Gastrointestinaltraktes, vorzugsweise Darmtumor oder Darmkrebs, wobei eine Bestimmung an mindestens einem Patienten mittels dem neuen Biomarker lösliches Cadherin-17 - ein proteolytisches Spaltprodukt des Cadherin-17 mit der SEQ ID No. 1 oder SEQ ID No. 2 erfolgt.

Cadherine (englisch "Calcium adhering", wie Ca-adherine) sind Kalziumionen abhängige transmembran Glykoproteine aus der Gruppe der Adhäsionsproteine. Sie kommen vorwiegend in Desmosomen und Adherens junctions vor und vermitteln Zellkontakte in verschiedenen Geweben. Die Cadherine spielen vor allem eine Rolle bei der Stabilisierung von Zell-Zellkontakten, der embryonalen Morphogenese, der Erhaltung der Zellpolarität und - differenzierung und der Signaltransduktion.

Beim Menschen wurden bis heute mehr als 80 Cadherine identifiziert. Gemeinsam sind allen Cadherinen mehrere extrazelluläre Cadherin-Domänen. Eine Cadherin-Domäne ist ca. 110 Aminosäuren lang, evolutiv sehr konserviert und besitzt negativ geladene Sequenzmotive, welche Kalziumionen-abhängige, homophile Bindungen vermitteln. Über kurze, ca. zehn Aminosäuren lange Linkersequenzen wiederholen sich diese ECs tandemartig zwischen 5- bis 34-mal, wobei die ECs beginnend am N-terminalen Ende durchnummeriert werden. Anhand dieser Anzahl der Cadherin-Domänen, ebenfalls an der cytoplasmatischen Domäne und der Größe eines Cadherins sowie an Gen-Clustern, wird die Superfamilie der Cadherine in sechs Gruppen unterteilt: die klassischen Cadherine, desmosomale Cadherine, Protocadherine, Proteinkinase-Cadherine, FAT-ähnliche Cadherine und 7-Transmembran-Cadherine. Auf die N-terminalen Cadherin-Domänen folgt eine einzelne Transmembrandomäne und schließlich eine intrazelluläre C-terminale Domäne. Die klassischen Cadherine mit E-cadherin als dem bekanntesten Vertreter sowie die desmosomalen Cadherine (Desmogleine, Desmocolline) besitzen fünf extrazelluläre Cadherin-Repeats und eine etwa 160 Aminosäuren lange hochkonservierte intrazelluläre Domäne. Diese zytoplasmatische Domäne spielt funktionell eine wichtige Rolle sowohl bei der Zelladhäsion als auch in Signalkaskaden. Über die Interaktion mit zytoplasmatischen Proteinen (Catenine bzw. Plakoglobin etc.) vermitteln die zytoplasmatischen Domänen der klassischen und desmosomalen Cadherine den engen Kontakt zum Aktin-Cytoskelett bzw. zu den intermediären Filamenten.

Die Expression von Cadherin-17 in kolorektalen Karzinomen ist bisher in zwei Arbeiten adressiert worden: Hinoi et al. [Hinoi T, Lucas PC, Kuick R, Hanash S, Cho KR, Fearon ER: CDX2 regulates liver intestine-cadherin expression in normal and malignant colon epithelium and intestinal metaplasia. Gastroenterology 2002, 123:1565-1577] berichten von einer weiterbestehenden hohen Expression von Cadherin-17 in 24 von 25 Karzinomen des differenzierten Typs. Ein Verlust der Cadherin-17 Expression im Verein mit dem Verlust der Cdx2 Expression findet sich bei den von der Arbeitsgruppe als LDMDC (large cell minimally differentiated carcinoma) bezeichneten Kolontumoren. Takamura et al. fanden in einem Kollektiv von 45 kolorektalen Tumoren eine auf Normalniveau erhaltene Cadherin-17 Expression in 62 % der Tumore [Takamura M, Ichida T, Matsuda Y, Kobayashi M, Yamagiwa S, Genda T, Shioji K, Hashimoto S, Nomoto M, Hatakeyama K, Ajioka Y, Sakamoto M, Hirohashi S, Aoyagi Y: Reduced expression of liver-intestine cadherin is associated with progression and lymph node metastasis of human colorectal carcinoma. Cancer Lett 2004, 212:253-259] und eine leicht reduzierte Expression in 17 Tumoren (38 %).

WO2005/110338 A offenbart ein Verfahren zur Diagnose, Prognose, Verlaufskontrolle und Risikostratifizierung von Tumor oder Krebs des Gastrointestinaltraktes, wobei jedoch eine Bestimmung auf Basis der extracellulären Domäne von Cadherin 17 (CDH 17) erfolgt, allerdings beschreibt dieses Dokument nicht das lösliche Cadherin 17.

Ko S. et al. "Overexpression of LI-cadherin in gastric cancer is associated with lymph node metastasis", BIOCHEMICAL AND BIOPHYSICAL RESEARCH COMMUNICATIONS, ACADEMIC PRESS INC. ORLANDO, FL, US, (2004) 562 - 568 beschreibt LI-Cadherin als Serumtumormarker für Magenkrebs, jedoch ohne lösliches Cadherin 17 zu offenbaren.

Lösliches Cadherin-17 als Biomarker für Krebs und Tumoren im Gastrointestinaltrakt, insbesondere Darmtumoren, ist im Stand der Technik nicht beschrieben.

Es ist zudem bereits bekannt, dass lösliches E-cadherin in Kulturüberständen von menschlichen Brustkrebszellen nachgewiesen worden ist (Damsky CH, Richa J, Solter D, Knudsen K, Buck CA: Identification and purification of a cell surface glycoprotein mediating intercellular adhesion in embryonic and adult tissue. Cell 1983, 34:455-466; Wheelock MJ, Buck CA, Bechtol KB, Damsky CH: Soluble 80-kd fragment of cell-CAM 120/80 disrupts cell-cell adhesion. J Cell Biochem 1987, 34:187-202).

In den folgenden Jahren wurden Nachweismethoden (überwiegend EIA and ELISA) für lösliches E-cadherin in Körperflüssigkeiten wie Blut und Urin entwickelt. Tatsächlich ist lösliches E-cadherin bereits im Serum von gesunden Kontrollpersonen nachweisbar. Eine signifikante Erhöhung der Konzentration löslichen E-cadherins wurde bei Patienten mit Tumoren u.a. auch des Kolorektums beschrieben. Da E-cadherin per se in Tumoren nicht überexprimiert ist (teilweise in der Expressionshöhe sogar reduziert), wird diese Erhöhung der Serumspiegel von löslichem E-cadherin der verstärkten proteolytischen Aktivität im Rahmen des karzinogenen Prozesses zugeschrieben. Weiterhin könnten zelluläre und histologische Veränderungen im Verlauf der Karzinogenese (Auflösung von interzellulären Kontaken und Zell-Matrix Adhäsion, Auflösen der Basalmembran) zu einem veränderten Transport und Stoffwechsel der Ektodomänen führen (Charalabopoulos K, Gogali A, Dalavaga Y, Daskalopoulos G, Vassiliou M, Bablekos G, Karakosta A, Constantopoulos S: The clinical significance of soluble E-cadherin in nonsmall cell lung cancer. Exp Oncol 2006, 28:83-85; Chan AO, Chu KM, Lam SK, Cheung KL, Law S, Kwok KF, Wong WM, Yuen MF, Wong BC: Early prediction of tumor recurrence after curative resection of gastric carcinoma by measuring soluble E-cadherin. Cancer 2005, 104:740-746; Wilmanns C, Grossmann J, Steinhauer S, Manthey G, Weinhold B, Schmitt-Graff A, von Specht BU: Soluble serum E-cadherin as a marker of tumour progression in colorectal cancer patients. Clin Exp Metastasis 2004, 21:75-78; Gofuku J, Shiozaki H, Doki Y, Inoue M, Hirao M, Fukuchi N, Monden M: Characterization of soluble E-cadherin as a disease marker in gastric cancer patients. Br J Cancer 1998, 78:1095-1101, Katayama M, Hirai S, Kamihagi K, Nakagawa K, Yasumoto M, Kato I: Soluble E-cadherin fragments increased in circulation of cancer patients. Br J Cancer 1994, 69:580-585).

Nachteilig an E-cadherin ist jedoch, dass dieser Marker nicht gewebespezifisch ist. Vielmehr wird E-cadherin in zahlreichen Epithelien unterschiedlicher Organe exprimiert; lösliches E-cadherin ist gar in erheblicher Konzentration bereits im Serum von gesunden Kontrollen vorhanden und wird bei verschiedenen Tumorentitäten in erhöhter Konzentration im Serum gemessen.

Es besteht jedoch ein hohes Bedürfnis Biomarker bereitzustellen, die eine hohe Sensitivität und Spezifität sowie eine organspezifische Selektivität aufweisen, so dass eine sichere Diagnose möglich ist.

Eine der Erfindung zugrunde liegende Aufgabe besteht daher darin, ein Verfahren zur Diagnose und Risikostratifizierung von Tumor oder Krebs des Gastrointestinaltraktes, insbesondere Darmtumor oder Darmkrebs, zu entwickeln, das eine verbesserte frühzeitige Diagnose ermöglicht.

Die Aufgabe wird durch ein Verfahren zur *in vitro* Diagnose und Risikostratifizierung von Tumor oder Krebs des Gastrointestinaltraktes, insbesondere Darmtumor oder Darmkrebs gelöst, wobei eine Bestimmung mittels dem neuen Biomarker sCadherin-17 und zwar das lösliche Cadherin-17 (proteolytisches Spaltprodukt) mit der SEQ ID No. 1 oder SEQ ID No. 2 erfolgt (im Folgenden "sCadherin-17" oder "lösliches Cadherin-17" genannt).

Erfindungsgemäß bevorzugt ist jedoch die *in vitro* Diagnose und Risikostratifizierung von Darmtumor oder Darmkrebs, wobei eine Bestimmung mittels dem erfindungsgemäßen Biomarker sCadherin-17 (proteolytisches Spaltprodukt) mit der SEQ ID No. 1 oder SEQ ID No. 2 erfolgt, da eine hohe Selektivität und Signifikanz erreicht werden kann.

Im Unterschied zu E-cadherin zeichnet sich sCadherin-17 durch eine außerordentlich hohe Gewebespezifität für das Darmepithel aus. Ferner weisen Tumore durch erhöhte und/oder veränderte proteolytische Aktivität ein verändertes "Ektodomänen-Shedding" von sCadherin-17 gegenüber normalen Darmepithelzellen auf. Daher ist sCadherin-17 ein geeigneter spezifischer Biomarker und vorzugsweise selektiv für Darmtumor oder Darmkrebs.

Ferner weist Cadherin-17 im Unterschied zu den anderen Cadherinen sieben Cadherin-Repeats, jedoch nur eine sehr kurze charakteristische zytoplasmatische Domäne von 21 Aminosäuren Länge auf; daher ist der Größenunterschied zwischen dem Transmembranprotein und der löslichen Form im Unterschied zu E-cadherin gering. Interagierende zytoplasmatische Proteine sind nicht bekannt; trotzdem ist sCadherin-17 offenbar allein durch die Interaktion der extrazellulären Domänen funktionell aktiv in der interzellulären Adhäsion. Die Sequenzhomologie von Cadherin-17 mit anderen Cadherinen beträgt insgesamt nur 20-30 %.

Die subzelluläre Lokalisation von Cadherin-17 unterscheidet sich von den anderen Cadherinen, die in hochstrukturierten Zellkontakten (junctional complexes) konzentriert sind. Cadherin-17 ist jedoch über die laterale Oberfläche der Zellen verteilt und nicht mit dem Zytoskelett assoziiert, so dass Cadherin-17 eine vorteilhafte hohe laterale Mobilität innerhalb der Zytoplasmamembran aufweist.

Im Rahmen dieser Erfindung wird unter "sCadherin-17" ein Spaltprodukt gemäß SEQ ID No. 1 oder SEQ ID No. 2 verstanden. Erfindungsgemäß umfasst ist daher das Spaltprodukt (SEQ ID No. 1 oder SEQ ID No. 2) , nämlich das so genannte "lösliche Cadherin-17". Diese Spaltung der extrazellulären Domäne (= Spaltprodukte) eines Transmembranproteins wird als "Ectodomänen-Shedding" bezeichnet. Das verbleibende Transmembranpeptid umfasst die Aminosäuren 786 bis 808 der SEQ ID No. 3 oder SEQ ID No. 4.

Daher betrifft die Erfindung auch solche Aminosäure-Sequenzen (Polypeptide, Proteine), die eine Sequenzidentität oder Homologie von 70% und mehr, vorzugsweise von 80% und mehr, besonders bevorzugt von 90-95% und mehr mit der SEQ ID No. 1 oder SEQ ID No. 2 aufweisen. Ebenfalls mit eingeschlossen sind solche analoge Aminosäure-Sequenzen, die aufgrund des Austausches von einer oder mehreren Aminosäure(n) in diesen Sequenzen, dennoch die gewünschte Funktion des erfindungsgemäßen Biomarkers zur *in vitro* Diagnose von Tumor oder Krebs des Gastrointestinaltraktes, insbesondere Darmtumor oder Darmkrebs gewährleisten (nachstehend in ihrer Gesamtheit "erfindungsgemäße Biomarker"). Der erfindungsgemäße Biomarker wird im Zellüberstand (in vitro) nachgewiesen .

Der Begriff "Tumor oder Krebs des Gastrointestinaltraktes" umfasst solchen Tumor oder Krebs der Organe im Magen-Darm-Trakt, wie Galle, einschließlich Gallengang, Gallenblase, Pankreas, Magen und Darm, insbesondere Dünn-, Dick-, Mastdarm.

Ebenfalls umfasst sind solche Formen wie Karzinom oder Adenome, ferner maligne oder benigne Tumoren, insbesondere kolorektale Tumore diverser Stadien im gesamten Intestinaltrakt.

Der erfindungsgemäße Biomarker konnte mittels einer differentiellen Proteomanalyse identifiziert werden. Hierzu wurden Proteine aus konditionierten Medien von a) gut differenzierten intestinalen Epithelzellen (Adenom) und b) undifferenzierten Karzinomzellen isoliert und aufkonzentriert. Die Proteine wurden mit einem Farbstoff gelabelt und einer 2D-Gelelektrophorese unterworfen mit einer isoelektrischen Fokussierung in der ersten und einer SDS-Gelelektrophorese in der zweiten Dimension.

Die differentielle Darstellung (differenzierte frühe Tumorzelle/undifferenzierte Karzinomzellen) ist in den Beispielen dargelegt und zeigt die spezifische Anreicherung von sCadherin-17 im Sekretom von Tumorzellen.

Die weitere Auswertung erfolgte mit Hilfe von LC-ESI-MS(/MS) (Flüssigchomatographie-Elektrosprayionisations-Massenspektrometrie). Dabei wurden zunächst die Proteine im Gel, in dem die Proben zuvor aufgetrennt wurden, mit Hilfe von Trypsin in einzelne Peptidfragmente zerlegt. Diese wurden mit Hilfe von Reversed-Phase HPLC voneinander separiert und massenspektrometrisch untersucht, um die einzelnen Peptide zu identifizieren. Selbstverständlich können hierbei auch andere geeignete massenspektrometrische Verfahren angewandt werden, beispielsweise MALDI-TOF-MS.

Die Erfindung betrifft ebenfalls die Identifizierung und Stratifizierung von Patienten mit erhöhtem Risiko oder/und einer ungünstigen Prognose eines Tumor oder Krebs des Gastrointestinaltraktes, insbesondere Darmtumor oder Darmkrebs, insbesondere bei symptomatischen und / oder asymptomatischen Patienten.

Nachdem Nachweis des erfindungsgemäßen Biomarkers sCadherin-17 können sich weitere Untersuchungen anschließen, wie z.B. eine Darmspiegelung (Koloskopie) oder bildgebende Verfahren, wie unten beschrieben, so dass bereits eine Früherkennung eines Darmtumors und / oder Darmkrebs erfolgen kann.

Das erfindungsgemäße Verfahren ermöglicht daher klinische Entscheidungen, die zu einem schnellen Therapieerfolg und zur Vermeidung von Todesfällen führen, solche wie ein chirurgischer Eingriff oder endoskopische Abtragung des erkrankten Gewebes. Solche klinische Entscheidungen umfassen ebenfalls weiterführende Behandlung mittels Arzneimitteln zur Behandlung oder Therapie von Tumor oder Krebs des Gastrointestinaltraktes, insbesondere Darmtumor oder Darmkrebs, wobei eine Bestimmung von sCadherin 17 (wie z.B. SEQ ID No. 1 oder SEQ ID No. 2) von einem zu untersuchenden Patienten erfolgt.

Daher betrifft die Erfindung ebenfalls ein Verfahren zur *in vitro* Diagnose von Patienten mit von Tumor oder Krebs des Gastrointestinaltraktes, insbesondere Darmtumor oder Darmkrebs, zur Durchführung von klinischen Entscheidungen, wie weiterführende Behandlung und Therapie mittels Arzneimitteln.

In einer weiteren bevorzugten Ausführungsform des erfindungsgemäßen Verfahrens erfolgt daher die *in vitro* Diagnose zur Prognose, zur differentialdiagnostischen Früherkennung und Erkennung, zur Beurteilung des Schweregrades und zur therapiebegleitenden Verlaufsbeurteilung von Tumor oder Krebs des Gastrointestinaltraktes, insbesondere Darmtumor oder Darmkrebs.

In einer weiteren bevorzugten Ausführungsform betrifft die Erfindung ein Verfahren zur *in vitro* Diagnostik zur Früh- oder Differentialdiagnose oder Prognose von Tumor oder Krebs des Gastrointestinaltraktes, insbesondere Darmtumor oder Darmkrebs, wobei eine Bestimmung des erfindungsgemäßen Biomarkers von einem zu untersuchenden Patienten durchgeführt wird.

In dem erfindungsgemäßen Verfahrenes erfolgt die Diagnose *in vitro* an einer dem zu untersuchenden Patienten zu vor entnommenen Gewebeprobe oder körperflüssigkeit (Blut, Plasma, Sekret, Urin); Aufgrund der Bestimmung der erfindungsgemäßen Biomarker wird eine hohe Signifikanz für den Krebs oder Tumor des Gastrointestinaltraktes, insbesondere Darmtumor oder Darmkrebs erzielt und anhand der vorhandenen Menge oder deren Veränderung (Levelierung: Erhöhung / Erniedrigung) in mindestens einer Patientenprobe kann die Diagnose erfolgen.

In einer weiteren Ausführungsform der Erfindung kann das erfindungsgemäße Verfahren im Rahmen einer in-vitro Diagnose mittels parallelen oder simultanen Bestimmungen des erfindungsgemäßen Biomarker durchgeführt werden (z.B. Multititerplatten mit 96 und mehr Kavitäten), wobei die Bestimmungen an mindestens einer Patientenprobe durchgeführt werden.

In einer weiteren Ausführungsform der Erfindung kann das erfindungsgemäße Verfahren mit Hilfe einer 2D-Elektrophorese erfolgen, wobei in der ersten Dimension eine isoelektrische Fokussierung, in der zweiten Dimension eine Gelelektrophorese durchgeführt wird (im weitesten Sinne ist hierzu die Proteomforschung ("Proteornics") anzuwenden).

In einer weiteren Ausführungsform kann das erfindungsgemäße Verfahren und dessen Bestimmungen mittels einem Schnelltest durchgeführt werden (z.B. lateral-flow Test), sei es in Einzel-oder Multiparameterbestimmung.

Ferner betrifft die Erfindung die Verwendung des erfindungsgemäßen Biomarker zur *in vitro* Diagnose und / oder Prognose und/oder zur Früh- oder Differentialdiagnose von Tumor oder Krebs des Gastrointestinaltraktes, insbesondere Darmtumor oder Darmkrebs.

Beschrieben werden bildgebende Verfahren , wie Sonographie, Endosonographie, Kontrast-Röntge, Angiographie, Ultraschall- Diagnostik, Tomographie (CT/MRCP/MRT), Kernspintomographie, Szintigraphie, Subtraktionsangiographie und Endoskopie.

Bei gegebener Nichtindikation auf Darmtumor oder Darmkrebs kann auf anderen Krebs und Tumor des Gastrointestinaltraktes mittels der erfindungsgemäßen Biomarker untersucht werden.

Verwendet wird eine entsprechende diagnostische Vorrichtung zur Durchführung der erfindungsgemäßen Verfahren.

Unter einer solchen diagnostischen Vorrichtung wird insbesondere ein Array oder Assay verstanden (z.B. Immunoassay, ELISA etc.), insbesondere ein Proteinbiochip (US6346413B1. US20050014292) im weitesten Sinne eine Vorrichtung zur Verwendung im erfindungsgemäßen Verfahren.

Verwendet wird ein Kit im erfindungsgemäßen Verfahren, insbesondere enthaltend Nachweisreagenzien und weitere Hilfsmittel. Solche Nachweisreagenzien umfassen z.B. Antikörper etc.

Der Nachweis und die Quantifizierung des erfindungsgemäßen Biomarker kann ebenfalls mit Hilfe weiterer, dem Fachmann geläufiger Protein-Diagnoseverfahren durchgeführt werden, insbesondere unter Verwendung radioaktiv oder fluoreszenzmarkierter Antikörper. Zu nennen sind hier insbesondere dazu geeignete bioanalytische Verfahren, wie zum Beispiel Western blot (1D und 2D), Immunhistochemie, Antikörperarrays, Luminex, ELISA, Immunfluoreszenz, Radioimmunoassays sowie weitere geeignete bioanalytische Verfahren, wie zum Beispiel massenspektrometrische Verfahren, z.B. MRM (Multi reaction monitoring) oder AQUA (absolute Quantification), mit deren Hilfe die Biomarker quantitativ gemessen werden können.

Nachfolgende Beispiele und Abbildungen dienen zur näheren Erläuterung der Erfindung, jedoch ohne die Erfindung auf diese Beispiele und Abbildungen zu beschränken.

### Beispiele und Abbildungen:

Zur Identifizierung von Biomarker-Kandidaten wurde das sogenannte Sekretom von kultivierten Tumorzellen verwendet. Das Sekretom umfasst nach erweiterter Definition alle Proteine, die über verschiedene Mechanismen von den Zellen in das Kulturmedium freigesetzt werden.

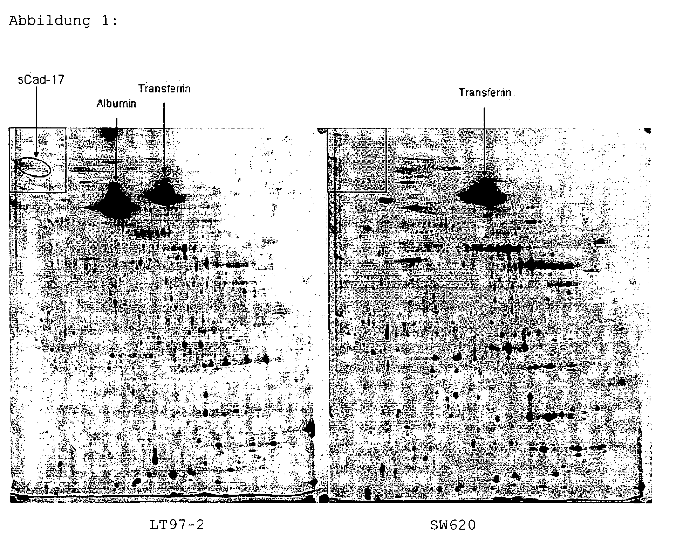

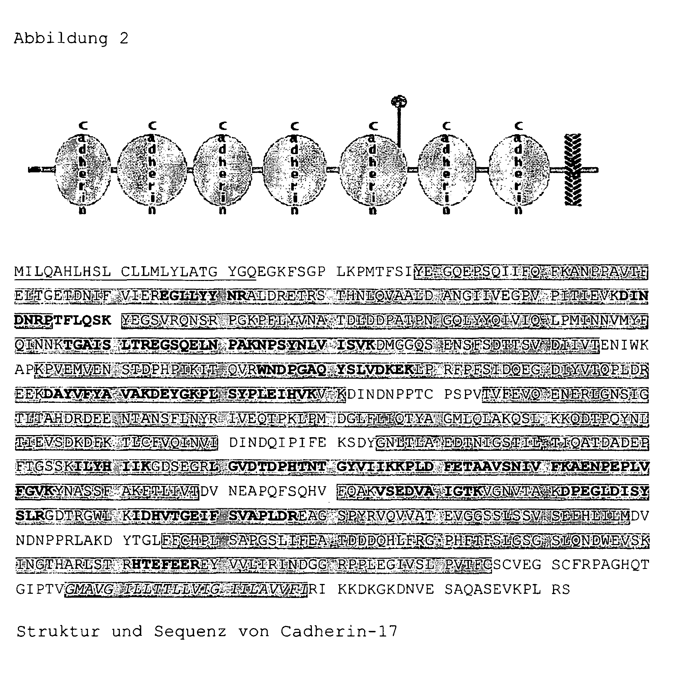

Hier wurden Sekretome von menschlichen SW620 Kolonkarzinomzellen (ATCC) und von menschlichen LT97-2 intestinalen Adenomzellen (bereitgestellt von Frau Prof. B. Marian, Wien) hergestellt wie beschrieben (Volmer MW, Radacz Y, Hahn SA, Klein-Scory S, Stuhler K, Zapatka M, Schmiegel W, Meyer HE, Schwarte-Waldhoff I: Tumor suppressor Smad4 mediates downregulation of the antiadhesive invasion-promoting matricellular protein SPARC: Landscaping activity of Smad4 as revealed by a "secretome" analysis. Proteomics 2004, 4:1324-1334; Volmer MW, Stuhler K, Zapatka M, Schoneck A, Klein-Scory S, Schmiegel W, Meyer HE, Schwarte-Waldhoff I: Differential proteome analysis of conditioned media to detect Smad4 regulated secreted biomarkers in colon cancer. Proteomics 2005, 5:2587-2601 und Diehl HC, Stühler K, Klein-Scory S, Volmer MW, Schöneck A, Bieling C, Schmiegel W, Meyer H, Schwarte-Waldhoff I: A catalogue of proteins released by colorectal cancer cells in vitro as an alternative source for biomarker discovery. Proteomics Clin. Appl. 2007, 1:47-61). Die Sekretome wurden mit CyDye Farbstoffen markiert, auf einem zweidimensionalen Gel aufgetrennt und die Proteinmuster mit dem Laser-Scanner eingelesen und dargestellt (Abb. 1). Prominente Spots des LT97-2 Sekretoms wurden aus dem Gel ausgestochen, tryptisch verdaut und durch Massenspektrometrie identifiziert wie beschrieben (Diehl et al., (supra)). Die in Abbildung 1 markierte Kette von Protein-Spots wurde als Cadherin-17 identifiziert. In der Abbildung 2 ist die Protein-Sequenz von Cadherin-17 dargestellt, die Cadherin-Domänen sind grau unterlegt (vgl. Schema); die identifizierten tryptischen Peptide sind durch Fettdruck markiert.

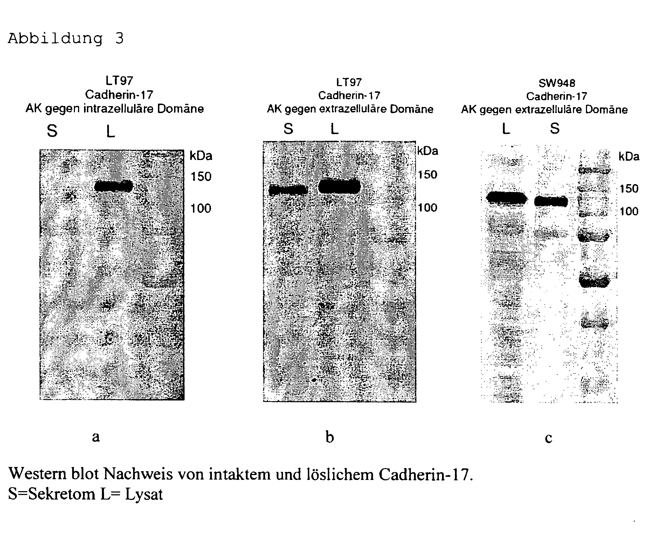

Cadherin-17 ist wie E-cadherin ein Transmembranprotein. Lösliches E-cadherin tritt im Sekretom durch Ektodomänen-Shedding auf, d.h. durch die Abspaltung und Freisetzung der extrazellulären Domänen. Durch Western blot Analysen von Sekretomen und Lysaten von LT97-2 Zellen wird in Abb. 3 gezeigt, dass lösliches Cadherin-17 ebenfalls durch Ektodomänen-Shedding gebildet wird und auf diese Art in das Sekretom gelangt: Zunächst wurde ein Antikörper verwendet, der gegen die C-terminale intrazelluläre Domäne von Cadherin-17 gerichtet ist (sc-6978, Santa Cruz, Affinitäts-aufgereinigter polyklonaler Antikörper, Ziege). Dieser Antikörper erkennt nur im Zelllysat ein Protein der erwarteten Größe (Abbildung 3a). Weiterhin wurde ein Antikörper verwendet, der gegen die extrazelluläre Domäne von Cadherin-17 gerichtet ist (141713, R&D, monoklonaler Antikörper aus der Maus). Dieser Antikörper erkennt im Zelllysat wiederum dasselbe intakte Transmembranprotein. Im Sekretom detektiert dieser Antikörper ein Protein, das geringfügig (ca. 10 kDa) kleiner ist: das lösliche Cadherin-17. Ein weiterer Antikörper gegen die extrazelluläre Domäne von Cadherin-17 (15-288-22817, GenWay, Affinitäts-aufgereinigter polyklonaler Antikörper, Huhn) bestätigte dieses Ergebnis (Abbildung 3b). Auch andere menschliche Darmtumorzellen zeigen das lösliche Cadherin-17 im Sekretom (Abbildung 3c).

### Erläuterung zu den Abbildungen:

### Abbildung 1:

Links ist das Sekretom von LT97-2 Zellen dargestellt, rechts das Sekretom von SW620 Zellen (in demselben Gel aufgetrennt, im Laser-Scanner getrennt eingelesen). Im LT-97-2 Sekretom ist die Spotkette markiert, die als Cadherin-17 identifiziert wurde. Ebenfalls markiert sind Transferrin und Albumin, die keine eigentlichen Sekretomkomponenten darstellen sondern Mediumzusatz bzw. Rest-Kontamination mit Albumin aus dem Vollmedium.

### Abbildung 2:

### Struktur und Sequenz von Cadherin-17

Das Signalpeptid ist in der Schemazeichnung links dunkel unterlegt, in der Sequenz unterstrichen. Die sieben Cadherin-Domänen sind grau unterlegt. Die Transmembrandomäne, in der Schemazeichnung mit "Reißverschlußmuster" gefüllt, ist in der Sequenz kursiv geschrieben. Die aus den in Abbildung 1 dargestellten Protein-Spots identifizierten Peptide sind fett gedruckt.

### Abbildung 3:

Western blot Nachweis von intaktem und löslichem Cadherin-17.

## Patentansprüche

1. Verfahren zur in-vitro Diagnose und Risikostratifizierung von Tumor oder Krebs des Gastrointestinaltraktes, insbesondere Darmtumor oder Darmkrebs, wobei eine Bestimmung von löslichem Cadherin 17 gemäß SEQ ID No. 1 oder SEQ ID No. 2 von einem zu untersuchenden Patienten erfolgt.

2. Verfahren zur in-vitro Diagnose und Risikostratifizierung nach Anspruch 1 zur Durchführung von klinischen Entscheidungen, insbesondere weiterführende Behandlung und Therapie mittels Arzneimitteln.

3. Verfahren zur in-vitro Diagnose und Risikostratifizierung nach einem der Ansprüche 1 bis 2, **dadurch gekennzeichnet, dass** die Diagnose zur Prognose, zur differentialdiagnostischen Früherkennung und Erkennung, zur Beurteilung des Schweregrades und zur therapiebegleitenden Verlaufsbeurteilung erfolgt.

4. Verfahren nach einem der vorherigen Ansprüche,
**dadurch gekennzeichnet,**
**dass** parallele oder simultane Bestimmungen des löslichen Cadherin 17 durchgeführt werden.

5. Verfahren nach einem der vorherigen Ansprüche, **dadurch gekennzeichnet, dass** eine 2D-Elektrophorese erfolgt, wobei in der ersten Dimension eine isoelektrische Fokussierung, in der zweiten Dimension eine Gelelektrophorese durchgeführt wird.

6. Verfahren nach einem der vorherigen Ansprüche,
**dadurch gekennzeichnet,**
**dass** die Bestimmungen an mindestens einer Patientenprobe durchgeführt werden.

7. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

8. Verwendung von löslichem Cadherin 17 gemäß SEQ ID No. 1 oder SEQ ID No. 2 zur in-vitro Diagnose und Risikostratifizierung von Tumor oder Krebs des Gastrointestinaltraktes, insbesondere Darmtumor, oder Darmkrebs.

9. Verwendung nach Anspruch 8, wobei die Bestimmung von löslichem Cadherin 17 gemäß SEQ ID No. 1 oder SEQ ID No. 2 mittels einem Kit enthaltend Nachweisreagenzien und weitere Hilfsmittel erfolgt, insbesondere in Form eines Array oder Assay.

## Claims

1. A method for in-vitro diagnosis and risk stratification of a tumor or cancer of the gastrointestinal tract, particularly colon tumor or colon cancer, wherein a determination of soluble cadherin 17 according to SEQ ID No. 1 or SEQ ID No. 2 is made for a patient to be examined.

2. A method for in-vitro diagnosis and risk stratification according to claim 1 for the execution of clinical decisions, particularly advanced treatment and therapy using drugs.

3. A method for in-vitro diagnosis and risk stratification according to one of the claims 1 to 2, **characterized in that** the diagnosis is made for prognosis, early detection and detection by differential diagnosis, assessment of the severity, and assessment of the course of the disease concomitant with the therapy.

4. A method according to any one of the preceding claims, **characterized in that** parallel or simultaneous determinations of soluble cadherin 17 are carried out.

5. A method according to any one of the preceding claims, **characterized in that** 2D electrophoresis is conducted, wherein isoelectric focusing is carried out in the first dimension and gel electrophoresis in the second dimension.

6. A method according to any one of the preceding claims, **characterized in that** the determinations are carried out using at least one patient sample.

7. A method according to any one of the preceding claims, **characterized in that** the determinations are carried out using a rapid test, particularly in individual or multiple parameter determinations.

8. Use of soluble cadherin 17 according to SEQ ID No. 1 or SEQ ID No. 2 for in-vitro diagnosis and risk stratification of a tumor or cancer of the gastrointestinal tract, particularly colon tumor or colon cancer

9. Use according to claim 8, wherein a determination of soluble cadherin 17 according to SEQ ID No. 1 or SEQ ID No. 2 is made by means of a kit comprising detection reagents and further media, particularly in the embodiment of an array or assay.

## Revendications

1. Méthode pour le diagnostic *in vitro* et la stratification du risque d'une tumeur ou d'un cancer du tractus gastro-intestinal, en particulier d'une tumeur du côlon ou d'un cancer du côlon, dans laquelle une détermination de la cadhérine 17 soluble conformément à SEQ ID No. 1 ou SEQ ID No. 2 est effectuée pour un patient à examiner.

2. Méthode pour le diagnostic *in vitro* et la stratification du risque selon la revendication 1 pour l'exécution de décisions cliniques, en particulier d'un traitement et d'une thérapie avancés à l'aide de médicaments.

3. Méthode pour le diagnostic *in vitro* et la stratification du risque selon l'une des revendications 1 à 2, **caractérisée par le fait que** le diagnostic est effectué pour un pronostic, une détection précoce et une détection par un diagnostic différentiel, une estimation de la sévérité et une estimation du déroulement de la maladie associé à la thérapie.

4. Méthode selon l'une quelconque des revendications précédentes, **caractérisée par le fait que** des déterminations parallèles ou simultanées de cadhérine 17 soluble sont réalisées.

5. Méthode selon l'une quelconque des revendications précédentes, **caractérisée par le fait qu'**une électrophorèse en 2D est réalisée, dans laquelle une électrofocalisation est réalisé dans la première dimension et une électrophorèse en gel dans la seconde dimension.

6. Méthode selon l'une quelconque des revendications précédentes, **caractérisée par le fait que** les déterminations sont réalisées à l'aide d'au moins un échantillon de patient.

7. Méthode selon l'une quelconque des revendications précédentes, **caractérisée par le fait que** les déterminations sont réalisées à l'aide d'un test rapide, en particulier dans des déterminations de paramètres individuels ou multiples.

8. Utilisation de la cadhérine 17 soluble conformément à SEQ ID No. 1 ou SEQ ID No. 2 pour un diagnostic *in vitro* et une stratification du risque d'une tumeur ou d'un cancer du tractus gastro-intestinal, en particulier d'une tumeur du côlon ou d'un cancer du côlon.

9. Utilisation selon la revendication 8, dans laquelle une détermination de la cadhérine 17 soluble conformément à SEQ ID No. 1 ou SEQ ID No. 2 est effectuée au moyen d'une trousse comprenant des réactifs de détection et d'autres moyens, en particulier dans le mode de réalisation consistant en un réseau ou un dosage.


### SEQUENCE LISTING

<110> Meyer et al., Helmut E.
<120> Lösliches Cadherin 17 für die Diagnose und Risikostratifizierung von Darmtumor oder Darmkrebs
<130> not yet known
<150> DE102007034993.0
   <151> 2007-07-26
<160> 4
<170> PatientIn version 3.3
<210> 1
   <211> 762
   <212> PRT
   <213> Human
<400> 1
   Glu Gly Lys Phe Ser Gly Pro Leu Lys Pro Met Thr Phe Ser Ile Tyr
   1               5                   10                  15
   Glu Gly Gln Glu Pro Ser Gln Ile Ile Phe Gln Phe Lys Ala Asn Pro
               20                  25                  30
   Pro Ala Val Thr Phe Glu Leu Thr Gly Glu Thr Asp Asn Ile Phe Val
           35                  40                  45
   Ile Glu Arg Glu Gly Leu Leu Tyr Tyr Asn Arg Ala Leu Asp Arg Glu
       50                  55                  60
   Thr Arg Ser Thr His Asn Leu Gln Val Ala Ala Leu Asp Ala Asn Gly
   65                  70                  75                  80
   Ile Ile Val Glu Gly Pro Val Pro Ile Thr Ile Glu Val Lys Asp Ile
                   85                  90                  95
   Asn Asp Asn Arg Pro Thr Phe Leu Gln Ser Lys Tyr Glu Gly Ser Val
               100                 105                 110
   Arg Gln Asn Ser Arg Pro Gly Lys Pro Phe Leu Tyr Val Asn Ala Thr
           115                 120                 125
   Asp Leu Asp Asp Pro Ala Thr Pro Asn Gly Gln Leu Tyr Tyr Gln Ile
       130                 135                 140
   Val Ile Gln Leu Pro Met Ile Asn Asn Val Met Tyr Phe Gln Ile Asn
   145                 150                 155                 160
   Asn Lys Thr Gly Ala Ile Ser Leu Thr Arg Glu Gly Ser Gln Glu Leu
                   165                 170                 175
   Asn Pro Ala Lys Asn Pro Ser Tyr Asn Leu Val Ile Ser Val Lys Asp
   180 185 190
   Met Gly Gly Gln Ser Glu Asn Ser Phe Ser Asp Thr Thr Ser Val Asp
   195 200 205
   Ile Ile Val Thr Glu Asn Ile Trp Lys Ala Pro Lys Pro Val Glu Met
   210 215 220
   Val Glu Asn Ser Thr Asp Pro His Pro Ile Lys Ile Thr Gln Val Arg
   225 230 235 240
   Trp Asn Asp Pro Gly Ala Gln Tyr Ser Leu Val Asp Lys Glu Lys Leu
   245 250 255
   Pro Arg Phe Pro Phe Ser Ile Asp Gln Glu Gly Asp Ile Tyr Val Thr
   260 265 270
   Gln Pro Leu Asp Arg Glu Glu Lys Asp Ala Tyr Val Phe Tyr Ala Val
   275 280 285
   Ala Lys Asp Glu Tyr Gly Lys Pro Leu Ser Tyr Pro Leu Glu Ile His
   290 295 300
   Val Lys Val Lys Asp Ile Asn Asp Asn Pro Pro Thr Cys Pro Ser Pro
   305 310 315 320
   Val Thr Val Phe Glu Val Gln Glu Asn Glu Arg Leu Gly Asn Ser Ile
   325 330 335
   Gly Thr Leu Thr Ala His Asp Arg Asp Glu Glu Asn Thr Ala Asn Ser
   340 345 350
   Phe Leu Asn Tyr Arg Ile Val Glu Gln Thr Pro Lys Leu Pro Met Asp
   355 360 365
   Gly Leu Phe Leu Ile Gln Thr Tyr Ala Gly Met Leu Gln Leu Ala Lys
   370 375 380
   Gln Ser Leu Lys Lys Gln Asp Thr Pro Gln Tyr Asn Leu Thr Ile Glu
   385 390 395 400
   Val Ser Asp Lys Asp Phe Lys Thr Leu Cys Phe Val Gln Ile Asn Val
   405 410 415
   Ile Asp Ile Asn Asp Gln Ile Pro Ile Phe Glu Lys Ser Asp Tyr Gly
   420 425 430
   Asn Leu Thr Leu Ala Glu Asp Thr Asn Ile Gly Ser Thr Ile Leu Thr
   435 440 445
   Ile Gln Ala Thr Asp Ala Asp Glu Pro Phe Thr Gly Ser Ser Lys Ile
   450 455 460
   Leu Tyr His Ile Ile Lys Gly Asp Ser Glu Gly Arg Leu Gly Val Asp
   465 470 475 480
   Thr Asp Pro His Thr Asn Thr Gly Tyr Val Ile Ile Lys Lys Pro Leu
   485 490 495
   Asp Phe Glu Thr Ala Ala Val Ser Asn Ile Val Phe Lys Ala Glu Asn
   500 505 510
   Pro Glu Pro Leu Val Phe Gly Val Lys Tyr Asn Ala Ser Ser Phe Ala
   515 520 525
   Lys Phe Thr Leu Ile Val Thr Asp Val Asn Glu Ala Pro Gln Phe Ser
   530 535 540
   Gln His Val Phe Gln Ala Lys Val Ser Glu Asp Val Ala Ile Gly Thr
   545 550 555 560
   Lys Val Gly Asn Val Thr Ala Lys Asp Pro Glu Gly Leu Asp Ile Ser
   565 570 575
   Tyr Ser Leu Arg Gly Asp Thr Arg Gly Trp Leu Lys Ile Asp His Val
   580 585 590
   Thr Gly Glu Ile Phe Ser Val Ala Pro Leu Asp Arg Glu Ala Gly Ser
   595 600 605
   Pro Tyr Arg Val Gln Val Val Ala Thr Glu Val Gly Gly Ser Ser Leu
   610 615 620
   Ser Ser Val Ser Glu Phe His Leu Ile Leu Met Asp Val Asn Asp Asn
   625 630 635 640
   Pro Pro Arg Leu Ala Lys Asp Tyr Thr Gly Leu Phe Phe Cys His Pro
   645 650 655
   Leu Ser Ala Pro Gly Ser Leu Ile Phe Glu Ala Thr Asp Asp Asp Gln
   660 665 670
   His Leu Phe Arg Gly Pro His Phe Thr Phe Ser Leu Gly Ser Gly Ser
   675 680 685
   Leu Gln Asn Asp Trp Glu Val Ser Lys Ile Asn Gly Thr His Ala Arg
   690 695 700
   Leu Ser Thr Arg His Thr Glu Phe Glu Glu Arg Glu Tyr Val Val Leu
   705 710 715 720
   Ile Arg Ile Asn Asp Gly Gly Arg Pro Pro Leu Glu Gly Ile Val Ser
   725 730 735
   Leu Pro Val Thr Phe Cys Ser Cys Val Glu Gly Ser Cys Phe Arg Pro
   740 745 750
   Ala Gly His Gln Thr Gly Ile Pro Thr Val
   755 760
<210> 2
   <211> 762
   <212> PRT
   <213> Human
<400> 2
   Glu Gly Lys Phe Ser Gly Pro Leu Lys Pro Met Thr Phe Ser Ile Tyr
   1               5                   10                  15
   Glu Gly Gln Glu Pro Ser Gln Ile Ile Phe Gln Phe Lys Ala Asn Pro
               20                  25                  30
   Pro Ala Val Thr Phe Glu Leu Thr Gly Glu Thr Asp Asn Ile Phe Val
           35                  40                  45
   Ile Glu Arg Glu Gly Leu Leu Tyr Tyr Asn Arg Ala Leu Asp Arg Glu
       50                  55                  60
   Thr Arg Ser Thr His Asn Leu Gln Val Ala Ala Leu Asp Ala Asn Gly
   65                  70                  75                  80
   Ile Ile Val Glu Gly Pro Val Pro Ile Thr Ile Glu Val Lys Asp Ile
                   85                  90                  95
   Asn Asp Asn Arg Pro Thr Phe Leu Gln Ser Lys Tyr Glu Gly Ser Val
               100                 105                 110
   Arg Gln Asn Ser Arg Pro Gly Lys Pro Phe Leu Tyr Val Asn Ala Thr
           115                 120                 125
   Asp Leu Asp Asp Pro Ala Thr Pro Asn Gly Gln Leu Tyr Tyr Gln Ile
       130                 135                 140
   Val Ile Gln Leu Pro Met Ile Asn Asn Val Met Tyr Phe Gln Ile Asn
   145                 150                 155                 160
   Asn Lys Thr Gly Ala Ile Ser Leu Thr Arg Glu Gly Ser Gln Glu Leu
                   165                 170                 175
   Asn Pro Ala Lys Asn Pro Ser Tyr Asn Leu Val Ile Ser Val Lys Asp
               180                 185                 190
   Met Gly Gly Gln Ser Glu Asn Ser Phe Ser Asp Thr Thr Ser Val Asp
           195                 200                 205
   Ile Ile Val Thr Glu Asn Ile Trp Lys Ala Pro Lys Pro Val Glu Met
       210                 215                 220
   Val Glu Asn Ser Thr Asp Pro His Pro Ile Lys Ile Thr Gln Val Arg
   225                 230                 235                 240
   Trp Asn Asp Pro Gly Ala Gln Tyr Ser Leu Val Asp Lys Glu Lys Leu
                   245                 250                 255
   Pro Arg Phe Pro Phe Ser Ile Asp Gln Glu Gly Asp Ile Tyr Val Thr
               260                 265                 270
   Gln Pro Leu Asp Arg Glu Glu Lys Asp Ala Tyr Val Phe Tyr Ala Val
           275                 280                 285
   Ala Lys Asp Glu Tyr Gly Lys Pro Leu Ser Tyr Pro Leu Glu Ile His
       290                 295                 300
   Val Lys Val Lys Asp Ile Asn Asp Asn Pro Pro Thr Cys Pro Ser Pro
   305                 310                 315                 320
   Val Thr Val Phe Glu Val Gln Glu Asn Glu Arg Leu Gly Asn Ser Ile
                   325                 330                 335
   Gly Thr Leu Thr Ala His Asp Arg Asp Glu Glu Asn Thr Ala Asn Ser
               340                 345                 350
   Phe Leu Asn Tyr Arg Ile Val Glu Gln Thr Pro Lys Leu Pro Met Asp
           355                 360                 365
   Gly Leu Phe Leu Ile Gln Thr Tyr Ala Gly Met Leu Gln Leu Ala Lys
       370                 375                 380
   Gln Ser Leu Lys Lys Gln Asp Thr Pro Gln Tyr Asn Leu Thr Ile Glu
   385                 390                 395                 400
   Val Ser Asp Lys Asp Phe Lys Thr Leu Cys Phe Val Gln Ile Asn Val
                   405                 410                 415
   Ile Asp Ile Asn Asp Gln Ile Pro Ile Phe Glu Lys Ser Asp Tyr Gly
               420                 425                 430
   Asn Leu Thr Leu Ala Glu Asp Thr Asn Ile Gly Ser Thr Ile Leu Thr
           435                 440                 445
   Ile Gln Ala Thr Asp Ala Asp Glu Pro Phe Thr Gly Ser Ser Lys Ile
       450                 455                 460
   Leu Tyr His Ile Ile Lys Gly Asp Ser Glu Gly Arg Leu Gly Val Asp
   465                 470                 475                 480
   Thr Asp Pro His Thr Asn Thr Gly Tyr Val Ile Ile Lys Lys Pro Leu
   485 490 495
   Asp Phe Glu Thr Ala Ala Val Ser Asn Ile Val Phe Lys Ala Glu Asn
   500 505 510
   Pro Glu Pro Leu Val Phe Gly Val Lys Tyr Asn Ala Ser Ser Phe Ala
   515 520 525
   Lys Phe Thr Leu Ile Val Thr Asp Val Asn Glu Ala Pro Gln Phe Ser
   530 535 540
   Gln His Val Phe Gln Ala Lys Val Ser Glu Asp Val Ala Ile Gly Thr
   545 550 555 560
   Lys Val Gly Asn Val Thr Ala Lys Asp Pro Glu Gly Leu Asp Ile Ser
   565 570 575
   Tyr Ser Leu Arg Gly Asp Thr Arg Gly Trp Leu Lys Ile Asp His Val
   580 585 590
   Thr Gly Glu Ile Phe Ser Val Ala Pro Leu Asp Arg Glu Ala Gly Ser
   595 600 605
   Pro Tyr Arg Val Gln Val Val Ala Thr Glu Val Gly Gly Ser Ser Leu
   610 615 620
   Ser Ser Val Ser Glu Phe His Leu Ile Leu Met Asp Val Asn Asp Asn
   625 630 635 640
   Pro Pro Arg Leu Ala Lys Asp Tyr Thr Gly Leu Phe Phe Cys His Pro
   645 650 655
   Leu Ser Ala Pro Gly Ser Leu Ile Phe Glu Ala Thr Asp Asp Asp Gln
   660 665 670
   His Leu Phe Arg Gly Pro His Phe Thr Phe Ser Leu Gly Ser Gly Ser
   675 680 685
   Leu Gln Asn Asp Trp Glu Val Ser Lys Ile Asn Gly Thr His Ala Arg
   690 695 700
   Leu Ser Thr Arg His Thr Asp Phe Glu Glu Arg Glu Tyr Val Val Leu
   705 710 715 720
   Ile Arg Ile Asn Asp Gly Gly Arg Pro Pro Leu Glu Gly Ile Val Ser
   725 730 735
   Leu Pro Val Thr Phe Cys Ser Cys Val Glu Gly Ser Cys Phe Arg Pro
   740 745 750
   Ala Gly His Gln Thr Gly Ile Pro Thr Val
   755 760
<210> 3
   <211> 832
   <212> PRT
   <213> Human
<400> 3
   Met Ile Leu Gln Ala His Leu His Ser Leu Cys Leu Leu Met Leu Tyr
   1               5                   10                  15
   Leu Ala Thr Gly Tyr Gly Gln Glu Gly Lys Phe Ser Gly Pro Leu Lys
               20                  25                  30
   Pro Met Thr Phe Ser Ile Tyr Glu Gly Gln Glu Pro Ser Gln Ile Ile
           35                  40                  45
   Phe Gln Phe Lys Ala Asn Pro Pro Ala Val Thr Phe Glu Leu Thr Gly
       50                  55                  60
   Glu Thr Asp Asn Ile Phe Val Ile Glu Arg Glu Gly Leu Leu Tyr Tyr
   65                  70                  75                  80
   Asn Arg Ala Leu Asp Arg Glu Thr Arg Ser Thr His Asn Leu Gln Val
                   85                  90                  95
   Ala Ala Leu Asp Ala Asn Gly Ile Ile Val Glu Gly Pro Val Pro Ile
               100                 105                 110
   Thr Ile Glu Val Lys Asp Ile Asn Asp Asn Arg Pro Thr Phe Leu Gln
           115                 120                 125
   Ser Lys Tyr Glu Gly Ser Val Arg Gln Asn Ser Arg Pro Gly Lys Pro
       130                 135                 140
   Phe Leu Tyr Val Asn Ala Thr Asp Leu Asp Asp Pro Ala Thr Pro Asn
   145                 150                 155                 160
   Gly Gln Leu Tyr Tyr Gln Ile Val Ile Gln Leu Pro Met Ile Asn Asn
                   165                 170                 175
   Val Met Tyr Phe Gln Ile Asn Asn Lys Thr Gly Ala Ile Ser Leu Thr
               180                 185                 190
   Arg Glu Gly Ser Gln Glu Leu Asn Pro Ala Lys Asn Pro Ser Tyr Asn
           195                 200                 205
   Leu Val Ile Ser Val Lys Asp Met Gly Gly Gln Ser Glu Asn Ser Phe
       210                 215                 220
   Ser Asp Thr Thr Ser Val Asp Ile Ile Val Thr Glu Asn Ile Trp Lys
   225 230 235 240
   Ala Pro Lys Pro Val Glu Met Val Glu Asn Ser Thr Asp Pro His Pro
   245 250 255
   Ile Lys Ile Thr Gln Val Arg Trp Asn Asp Pro Gly Ala Gln Tyr Ser
   260 265 270
   Leu Val Asp Lys Glu Lys Leu Pro Arg Phe Pro Phe Ser Ile Asp Gln
   275 280 285
   Glu Gly Asp Ile Tyr Val Thr Gln Pro Leu Asp Arg Glu Glu Lys Asp
   290 295 300
   Ala Tyr Val Phe Tyr Ala Val Ala Lys Asp Glu Tyr Gly Lys Pro Leu
   305 310 315 320
   Ser Tyr Pro Leu Glu Ile His Val Lys Val Lys Asp Ile Asn Asp Asn
   325 330 335
   Pro Pro Thr Cys Pro Ser Pro Val Thr Val Phe Glu Val Gln Glu Asn
   340 345 350
   Glu Arg Leu Gly Asn Ser Ile Gly Thr Leu Thr Ala His Asp Arg Asp
   355 360 365
   Glu Glu Asn Thr Ala Asn Ser Phe Leu Asn Tyr Arg Ile Val Glu Gln
   370 375 380
   Thr Pro Lys Leu Pro Met Asp Gly Leu Phe Leu Ile Gln Thr Tyr Ala
   385 390 395 400
   Gly Met Leu Gln Leu Ala Lys Gln Ser Leu Lys Lys Gln Asp Thr Pro
   405 410 415
   Gln Tyr Asn Leu Thr Ile Glu Val Ser Asp Lys Asp Phe Lys Thr Leu
   420 425 430
   Cys Phe Val Gln Ile Asn Val Ile Asp Ile Asn Asp Gln Ile Pro Ile
   435 440 445
   Phe Glu Lys Ser Asp Tyr Gly Asn Leu Thr Leu Ala Glu Asp Thr Asn
   450 455 460
   Ile Gly Ser Thr Ile Leu Thr Ile Gln Ala Thr Asp Ala Asp Glu Pro
   465 470 475 480
   Phe Thr Gly Ser Ser Lys Ile Leu Tyr His Ile Ile Lys Gly Asp Ser
   485 490 495
   Glu Gly Arg Leu Gly Val Asp Thr Asp Pro His Thr Asn Thr Gly Tyr
   500 505 510
   Val Ile Ile Lys Lys Pro Leu Asp Phe Glu Thr Ala Ala Val Ser Asn
   515 520 525
   Ile Val Phe Lys Ala Glu Asn Pro Glu Pro Leu Val Phe Gly Val Lys
   530 535 540
   Tyr Asn Ala Ser Ser Phe Ala Lys Phe Thr Leu Ile Val Thr Asp Val
   545 550 555 560
   Asn Glu Ala Pro Gln Phe Ser Gln His Val Phe Gln Ala Lys Val Ser
   565 570 575
   Glu Asp Val Ala Ile Gly Thr Lys Val Gly Asn Val Thr Ala Lys Asp
   580 585 590
   Pro Glu Gly Leu Asp Ile Ser Tyr Ser Leu Arg Gly Asp Thr Arg Gly
   595 600 605
   Trp Leu Lys Ile Asp His Val Thr Gly Glu Ile Phe Ser Val Ala Pro
   610 615 620
   Leu Asp Arg Glu Ala Gly Ser Pro Tyr Arg Val Gln Val Val Ala Thr
   625 630 635 640
   Glu Val Gly Gly Ser Ser Leu Ser Ser Val Ser Glu Phe His Leu Ile
   645 650 655
   Leu Met Asp Val Asn Asp Asn Pro Pro Arg Leu Ala Lys Asp Tyr Thr
   660 665 670
   Gly Leu Phe Phe Cys His Pro Leu Ser Ala Pro Gly Ser Leu Ile Phe
   675 680 685
   Glu Ala Thr Asp Asp Asp Gln His Leu Phe Arg Gly Pro His Phe Thr
   690 695 700
   Phe Ser Leu Gly Ser Gly Ser Leu Gln Asn Asp Trp Glu Val Ser Lys
   705 710 715 720
   Ile Asn Gly Thr His Ala Arg Leu Ser Thr Arg His Thr Glu Phe Glu
   725 730 735
   Glu Arg Glu Tyr Val Val Leu Ile Arg Ile Asn Asp Gly Gly Arg Pro
   740 745 750
   Pro Leu Glu Gly Ile Val Ser Leu Pro Val Thr Phe Cys Ser Cys Val
   755 760 765
   Glu Gly Ser Cys Phe Arg Pro Ala Gly His Gln Thr Gly Ile Pro Thr
   770 775 780
   Val Gly Met Ala Val Gly Ile Leu Leu Thr Thr Leu Leu Val Ile Gly
   785 790 795 800
   Ile Ile Leu Ala Val Val Phe Ile Arg Ile Lys Lys Asp Lys Gly Lys
   805 810 815
   Asp Asn Val Glu Ser Ala Gln Ala Ser Glu Val Lys Pro Leu Arg Ser
   820 825 830
<210> 4
   <211> 832
   <212> PRT
   <213> Human
<400> 4